# EUROPEAN PATENT SPECIFICATION

(11) **EP 0 795 329 B1**
(45) Date of publication and mention of the grant of the patent: **31.01.2001**
(21) Application number: 97830103.4
(22) Date of filing: 07.03.1997
(51) Int. Cl.: A61K 31/54, A61K 9/08, A61K 47/18

(54) **Parenterally injectable piroxicam solutions**
Parenteral injizierbare Lösungen enthaltend Piroxicam
Solutions parentérales injectables contenant le piroxicam

(30) Priority: 15.03.1996 IT MI960501
(43) Date of publication of application: 17.09.1997
(73) Proprietor: PULITZER ITALIANA S.r.l., 00156 Roma (IT)
(72) Inventor: Bertone, Evaristo, 00156 Roma (IT)
(74) Representative: Minoja, Fabrizio, Dr.

(56) References cited:
- EP-A- 0 066 458
- EP-A- 0 066 459
- EP-A- 0 093 999
- EP-A- 0 177 870
- EP-A- 0 336 200
- DE-A- 3 700 172
- US-A- 5 420 124
- DATABASE WPI Week 9210 Derwent Publications Ltd., London, GB; AN 92-075196 [30] XP002033526 & JP 04 018 022 A (FUJIKAWA KK) 22 January 1992
- DATABASE WPI Week 9312 Derwent Publications Ltd., London, GB; AN 93-098122 [12] XP002033527 & KR 9 206 911 A (SHIN POONG PHARM. CO.) 22 August 1992

## Description

The present invention relates to injectable pharmaceutical formulations containing Piroxicam.

Piroxicam is a non steroidal antiinflammatory agent which is widely used in clinic, mainly by the oral route.

On the contrary, the preparation of a pharmaceutical form for the parenteral use is hampered by the poor water-solubility of Piroxicam. A number of solutions to this problem have been proposed, none of them being fully satisfactory.

USA Patent 4,233,299 discloses the salification of many oxicam derivatives with methylglucamine.

However, the Piroxicam aqueous solutions obtained by salification proved to be physically unstable resulting in the precipitation of the active ingredient in time.

DE Patent 3,217,315 describes Piroxicam solutions obtained using both methylglucamine as the salifying agent, in proportions even higher than the stoichiometric ones, and at the same time a water-miscibile organic solvent (propylene glycol). Such solutions are stated to have a good physical stability (absence of precipitates).

DE Patent 3,437,232 (U.S. 4,628,053) showed that Piroxicam solutions, in aqueous-organic solvent, in the absence of methylglucamine, although having a good physical stability, undergo chemical degradation of the active ingredient due to hydrolysis, thus releasing 2-aminopyridine. The same Patent discloses that sub-stoichiometric doses of methylglucamine (20-90% of theoretical) are sufficient to stabilize the Piroxicam solution in an aqueous solvent containing propylene glycol (40% v/v) and ethyl alcohol (10% v/v), buffered at a pH from 8 to 9.

Now it has been found that Piroxicam solutions in a mixture of water, propylene glycol, polyethylene glycol and ethyl alcohol are stabilized both physically (absence of precipitates) and chemicallly (absence of hydrolysis of the active ingredient) adding tromethamine (TRIS buffer) in amounts so as to salify 40-60% of Piroxicam and at the same time sufficient to buffer the solution to a final pH of 8-9.

It has also been found that the above described solutions may be added with benzyl alcohol and, above all, lidocaine, although it is nearly insoluble at pH 8-9, in amounts effective respectively as an bacteriostatic and as a local anaesthetic, without incompatibilities, precipitations or decrease in stability taking place.

The present invention therefore discloses novel Piroxicam solutions comprising tromethamine and optionally lidocaine and benzyl alcohol in an aqueous organic solvent and process for the preparation thereof.

Said solutions are stable and, notwithstanding the very high pH, suitable for the intramuscular injection without causing pain or burning sensation. The compositions of the invention are moreover suitable to be sterilized by membrane sterilization and distributed in vials in aseptic conditions.

Tromethamine or TRIS, in addition to the known buffering and alkalinizing effects, also exerts a stabilizing effect and it can replace methylglucamine in the preparations of Piroxicam for the parenteral use even in sub-molar dosages compared with Piroxicam.

The compositions of the invention are characterized by the following composition:

| | |
|---|---|
| Piroxicam | 1-6 % w/v; |
| Propylene glycol | 20-28% v/v; |
| Polyethylene glycol (100-400) | 12-20% v/v; |
| Ethanol | 3-6 %; |
| Tromethamine | 0.4 to 0.6 moles per mole of Piroxicam; |
| Water | 40-60% up to 100%. |

Preferably, the compositions of the invention will contain also a local anaesthetic and/or bacteriostatic agent.

As a local anaesthetic, lidocaine can be used in percentages varying from 0.5 to 3% w/v, which can be added to the solution in the form of the hydrochloride from which the free base is released by addition of a stoichiometric amount of sodium hydroxide. As a bacteriostatic agent, benzyl alcohol is particularly preferred in percentages varying from 1 to 3% w/v.

The compositions of the invention will contain preferably 2 to 4% w/v of Piroxicam and 0.5 to 0.6 moles of tromethamine per mole of Piroxicam.

The compositions of the invention are prepared mixing successively glycol, polyethylene glycol and ethyl alcohol, then adding Piroxicam and stirring until dissolution. After that, the tromethamine water solution and then sodium hydroxide to pH 8-9 are added, mixing until dissolution. Afterwards, if desired, lidocaine hydrochloride, benzyl alcohol and the amount of sodium hydroxide (10% solution) necessary to release lidocaine are added, stirring thoroughly. pH is adjusted to 8-9 by means of 10% sodium hydroxide or hydrochloric acid solutions, adjusting to the desired volume.

The resulting Piroxicam solution is sterilized by membrane filtration under nitrogen pressure and then partitioned into vials in aseptic conditions.

In this preparation method, the possible hydrolytic degradation of Piroxicam with formation of 2-aminopyridine has been taken into account. In fact, the salification is carried out in the hydro-organic medium of the specific solvent and prolonged permanence times of the active ingredient at a high pH are avoided. The compositions according to the invention have a final pH ranging from 8 to 9 and satisfy the stability (challenge test 5°-25°C) and storing tests (25°C and room temperature).

### Examples.

The following examples illustrate in more detail the present invention.

### Example 1

Following the procedure described above, a Piroxicam injectable solution having the following composition is prepared:
- Piroxicam 20 g
- Propylene glycol 280 ml
- Polyethylene glycol 200 120 ml
- Ethyl alcohol 60 ml
- Tromethamine 4 g
- Lidocaine hydrochloride 10 g
- Benzyl alcohol 15 g
- Sodium hydroxide 3.1 g
- Sterile depurated water q.s. to ml 1000.

The solution is stable and when administered intramuscularly, it causes neither pain nor burning sensation and is well tolerated topically.

### Example 2

Following the procedure described above, a Piroxicam injectable solution having the following composition is prepared:
- Piroxicam 20 g
- Propylene glycol 270 ml
- Polyethylene glycol 400 130 ml
- Ethyl alcohol 60 ml
- Tromethamine 4 g
- Lidocaine hydrochloride 10 g
- Benzyl alcohol 15 g
- Sodium hydroxide 3.1 g
- Sterile depurated water q.s. to ml 1000.

The solution is stable and when administered intramuscularly, it causes neither pain nor burning sensation and is well tolerated topically.

## Claims

1. An injectable solution of Piroxicam comprising the following:
| | |
|---|---|
| Piroxicam | 1-6 % w/v; |
| Propylene glycol | 20-28% v/v; |
| Polyethylene glycol (100-400) | 12-20% v/v; |
| Ethanol | 3-6 %; |
| Tromethamine | 0.4 to 0.6 moles per mole of Piroxicam; |
| Water | 40-60% up to 100%. |

2. A solution according to claim 1 wherein the solvent has the following composition: 28% v/v propylene glycol, 12% v/v polyethylene glycol 200 or 400, 6% v/v ethyl alcohol and 54% v/v water.

3. A solution according to claim 1, wherein Piroxicam is present in a concentration ranging from 2 to 4% w/v.

4. A solution according to claim 1, wherein tromethamine is present in an amount of 0.5-0.6 moles per mole of Piroxicam and pH ranges from 8 to 9.

5. A stable solution according to any one of the above claims, further comprising 0.5 to 3% w/v lidocaine hydrochloride as a local anaesthetic and 1 to 3% w/v benzyl alcohol as a bacteriostatic, and with a pH ranging from 8 to 9.

6. A solution according to claim 5 containing 2% w/v Piroxicam, 0.55 moles of tromethamine per mole of Piroxicam, and with a pH ranging from 8 to 8.5.

## Patentansprüche

1. Eine injizierbare Piroxam-Lösung, umfassend
| | |
|---|---|
| Piroxicam | 1-6 % w/v; |
| Propylenglykol | 20-28 % v/v; |
| Polyethylenglykol (100-400) | 12-20 % v/v; |
| Ethanol | 3-6 %; |
| Tromethamin | 0,4 bis 0,6 mol pro Mol Piroxicam; |
| Wasser | 40-60 % bis 100 %. |

2. Lösung nach Anspruch 1, wobei das Lösungsmittel die nachstehende Zusammensetzung hat: 28 % v/v Propylenglykol, 12 % v/v Polyethylenglykol 200 oder 400, 6 % v/v Ethylalkohol und 54 % v/v Wasser.

3. Lösung nach Anspruch 1, wobei Piroxicam in einer Konzentration im Bereich von 2 bis 4 % w/v vorliegt.

4. Lösung nach Anspruch 1, wobei Tromethamin in einer Menge von 0,5-0,6 mol pro Mol Piroxicam vorliegt und der pH-Wert im Bereich von 8 bis 9 liegt.

5. Eine stabile Lösung nach einem der vorstehenden Ansprüche, die weiter 0,5 bis 3 % w/v Lidocainhydrochlorid als Lokalanästhetikum und 1 bis 3 % w/v Benzylalkohol als Bakteriostatikum umfasst sowie einen pH-Wert im Bereich von 8 bis 9 aufweist.

6. Lösung nach Anspruch 5, die 2 % w/v Piroxicam und 0,55 mol Tromethamin pro Mol Piroxicam enthält sowie einen pH-Wert im Bereich von 8 bis 8,5 aufweist.

## Revendications

1. Solution injectable de Piroxicam comprenant ce qui suit :
| | |
|---|---|
| Piroxicam | 1 à 6 % p/v ; |
| Propylèneglycol | 20 à 28 % v/v ; |
| Polyéthylèneglycol (100-400) | 12 à 20 % v/v ; |
| Ethanol | 3 à 6 % ; |
| Trométhamine | 0,4 à 0,6 moles par mole de Piroxicam ; |
| Eau | 40 à 60 % jusqu'à 100 %. |

2. Solution selon la revendication 1, dans laquelle le solvant possède la composition suivante : 28 % v/v de propylèneglycol, 12 % v/v de polyéthylèneglycol 200 ou 400, 6 % v/v d'alcool éthylique et 54 % v/v d'eau.

3. Solution selon la revendication 1, dans laquelle le Piroxicam est présent en une concentration allant de 2 à 4 % p/v.

4. Solution selon la revendication 1, dans laquelle la trométhamine est présente à raison de 0,5 à 0,6 mole par mole de Piroxicam et le pH va de 8 à 9.

5. Solution stable selon l'une quelconque des revendications ci-dessus, comprenant de plus 0,5 à 3 % p/v de chlorhydrate de lidocaïne en tant qu'anesthésique local et 1 à 3 % p/v d'alcool benzylique en tant qu'agent bactériostatique, et avec un pH allant de 8 à 9.

6. Solution selon la revendication 5, contenant 2 % p/v de Piroxicam, 0,55 mole de trométhamine par mole de Piroxicam, et avec un pH allant de 8 à 8,5.
